# EUROPEAN PATENT SPECIFICATION

(11) **EP 2 026 787 B1**
(45) Date of publication and mention of the grant of the patent: **25.12.2013**
(21) Application number: 07728962.7
(22) Date of filing: 10.05.2007
(51) Int. Cl.: A61K 31/155, A61K 31/451, A61K 9/16, A61K 9/26, A61P 3/10

(54) **TABLET FORMULATION COMPRISING REPAGLINIDE AND METFORMIN**
REPAGLINID UND METFORMIN ENTHALTENDE TABLETTEN-FORMULIERUNG
FORMULATION DE COMPRIME COMPRENANT DU REPAGLINIDE ET DE LA METFORMINE

(30) Priority: 13.05.2006 EP 06009902; 09.10.2006 EP 06121953
(43) Date of publication of application: 25.02.2009
(73) Proprietor: Novo Nordisk A/S, 2880 Bagsværd (DK)
(72) Inventor: WADA, Koichi, Kawabe-gun, Hyogo 666-0257 (JP); FUJITA, Hikaru, Osaka-city, Osaka-pref. 534-0016 (JP); NAKATANI, Manabu, Kawanishi, Hyogo 666-0037 (JP); FRIEDL, Thomas, 88416 Ochsenhausen (DE)
(86) International application number: PCT/EP2007/054510
(87) International publication number: WO 2007/131930

(56) References cited:
- WO-A-98/56378
- WO-A-2007/056387
- US-A1- 2001 036 479
- US-A1- 2003 162 816
- US-A1- 2003 224 046
- US-A1- 2004 086 562
- US-A1- 2005 054 731
- US-A1- 2005 124 663

## Description

The present invention relates to a pharmaceutical fixed dose combination tablet comprising repaglinide and metformin. The present invention also provides a method of producing said tablet.

### BACKGROUND OF THE INVENTION

Metformin disclosed in US 3,174,901 is a long-acting biguanide antidiabetic that is mainly known for its antihyperglycaemic activity and is widely used in the treatment of non-insulin dependent diabetes mellitus (NIDDM). Its chemical name is *N,N*-dimethylimidodicarbon-imidic diamide having the following structure:

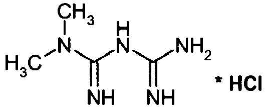

Metformin is manufactured and supplied as hydrochloride salt form.

Metformin is freely soluble in water (Martindale, 33rd Edition, page 332 (2002)). It is also known to be a poorly compressible substance. A poorly compressible substance is one that does not bind to form a tablet upon application of compression force. Therefore, such substances may require additional processing and special formulating before they can be compressed into tablets. With such substances, the additional processing necessary is usually a wet granulation step, as direct compression would not be effective. These substances may be formulated with binders or other materials that have high binding capacity (or that act as an aid to compressibility) such that the non-bonding properties of the non-compressible material are overcome. Other techniques to assist compression include having residual moisture in the blend prior to compression or having the non-compressible material in very low amounts in the tablet formulation. High-dose drugs, such as metformin, do not lend themselves to direct compression because of the relatively low proportion of diluent or compression aid in the tablet, poor powder flow and poor compressibility.

Repaglinide is i.a. disclosed in European patent application No. 0 589 874. It is a short-acting hypoglycaemic antidiabetic with the chemical name (S)-2-Ethoxy-4-[2-[[3-methyl-1-[2-(1-piperidinyl)phenyl]butyl]amino]-2-oxoethyl]benzoic acid having the following formula:

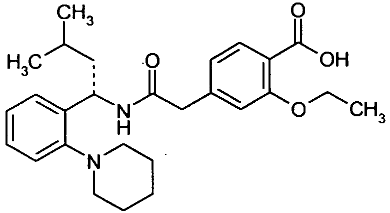

The solubility of the drug substance repaglinide is quite low (9 micro gram/ml in pH 5.0 buffer solution).

### OBJECTS OF THE INVENTION

The mechanisms of action of repaglinide and metformin are considered to cooperate favourable in the treatment of type I and II diabetes mellitus.

Combination therapy of repaglinide with metformin is expected to show synergistic therapeutic efficacy in the treatment of type I and type II diabetes mellitus. As this assumption gets supported by an increasing amount of clinical data, there is an increasing desire for a fixed dose combination drug comprising the active ingredients repaglinide and metformin.

It was therefore an object of the present invention to provide a fixed-dose combination drug comprising repaglinide and metformin, said combination drug displaying the fast dissolution and immediate drug release profile combined with adequate stability. Generally, a fixed-dose combination of drugs intended for immediate release is prepared by either making a powder mixture or a co-granulate of the two active ingredients with the necessary excipients, normally keeping the basic formulation of the corresponding mono-drug preparation and simply adding the second drug component.

With a combination of repaglinide and metformin, this approach was not feasible due to the fact that metformin has to be provided in a much higher quantity than repaglinide and due to the differences in solubility.

However, both repaglinide and metformin are chemical compounds difficult to handle. Therefore, an oral fixed dose combination drug which combines the features of pharmacologic efficacy, adequate drug stability and a reliable and robust method of manufacture has to overcome a number of technical problems. It is an object of the present invention to provide such a fixed dose combination drug.

It is an object of the present invention to provide a pharmaceutical composition that addresses the general challenges associated with the development of a pharmaceutical product, the specific challenges associated with the individual active compounds incorporated in the dosage form and also the challenges associated with bringing the active substances into combination.

A particular challenge associated with this combination is to ensure the bioequivalence of each active compound to the respective components when administered separately in spite of the biopharmaceutical problems associated with repaglinide and the different physical and chemical properties of both actives.

It is another object of the present invention to obtain a formulation of repaglinide and metformin with a size suitable for administration and acceptable to patients in spite of the fact that the composition of the invention shall contain a high amount of metformin (metformin is usually prescribed at 850 mg once or twice a day or at 500 mg three to four times a day). This considerable mass of metformin is to be combined in the same pharmaceutical dosage unit with repaglinide in smaller quantities than metformin (repaglinide is usually prescribed at 0.5 to 2 mg three to four times a day). Prior art teaches that such combination are associated with a large quantity of excipient in order to maintain an acceptable bioavailability (US 6,074,670), what would result in a large tablet.

A further object of the present invention is to obtain a formulation which gives rise to high patient compliance, by reducing the number of unit forms of administration that need to be taken, such as tablets. Diabetes mellitus type II often requires treatment with more than one active substance. In addition, amongst type II diabetes, the prevalence of other disorders associated with insulin resistance (dyslipidaemia, hypertension), which frequently require additional pharmacological forms of treatment, is high. Patient compliance under such circumstances is quite a problem, because individual dosage units are necessarily quite large in view of the high amounts of active substances which need to be administered, and the practical limits as regards the mass of pharmaceutical compositions which can be administered to a patient as a single dosage unit.

An even further object of the present invention is to provide a pharmaceutical composition containing both active components, repaglinide and metformin, whilst maintaining a bioavailability of each of the two components equivalent to or superior to that obtained with repaglinide alone or metformin alone. The object of the present invention is to obtain a formulation wherein both products are bioequivalent or suprabioavailable compared to bioavailability of monotherapy.

Another object of the present invention is to provide a process for preparing the pharmaceutical compositions fulfilling the objectives listed above, such processes being able to be accomplished with a limited number of different steps and being inexpensive.

### SUMMARY OF THE INVENTION

In accordance with the present invention, it has now been found that the solubility problem of the drug substance repaglinide could be overcome by using a granulate obtained by a spray drying (SD) process or by using the active triturate, which is a mixture of repaglinide SD granulate and microcrystalline cellulose. Repaglinide SD granulate is the spray dried granulate of the mixture of repaglinide, Poloxamer 188, Povidone K 25 and meglumine.

Metformin hydrochloride is highly soluble in water and drug load of metformin is more than 80% w/w of the composition, so this formulation is too sensitive to the amount of moistening water for high shear granulation. The granulates manufactured by high share granulation have poor compressibility.

Repaglinide and metformin fixed-dose tablets which have good content uniformity, fast dissolution and enough hardness have been developed. Improvement of the repaglinide's content uniformity was investigated intensively; this problem was solved by the co-granulation of repaglinde active triturate and metformin using the fluidized bed granulation technique. For the enough hardness of the tablet, fluidized bed granulation is needed whereas direct compression and high-share granulation are not effective for improving the tablet properties. The amount of binder and microcrystalline cellulose (MCC) are also important for the properties. The moisture content of the granulate also has a big impact on the hardness.

### DETAILED DESCRIPTION OF THE INVENTION

A first aspect of the present invention is a pharmaceutical tablet comprising repaglinide and metformin in a fast disintegrating tablet matrix [The term "disintegrating tablet matrix" refers to a pharmaceutical tablet base formulation having immediate release characteristics that readily swells and disintegrates in a physiological aqueous medium.] The tablet preferably contains repaglinide in substantially amorphous form. [The term "substantially amorphous" refers to repaglinide comprising amorphous constituents in a proportion of at least 90%, preferably at least 95%, as determined by X-ray powder diffraction measurement].

Substantially amorphous repaglinide may be produced by any suitable method known to those skilled in the art, for instance, by freeze drying of aqueous solutions, coating of carrier particles in a fluidized bed, and solvent deposition on sugar pellets or other carriers. Preferably, however, the substantially amorphous repaglinide is prepared by the specific spray-drying method described hereinafter.

The other active ingredient metformin is generally supplied in its free basic form, although pharmaceutically acceptable salts may also be used. Preferred is the metformin hydrochloride with a specific particle size distribution, which is usually employed as a fine-crystalline powder, optionally in fine-milled, peg-milled or micronized form. For instance, the particle size distribution of metformin hydrochloride in the tablet, as determined by the method of laser light scattering in a dry dispersion system (Sympatec Helos/Rodos, focal length 100 mm) is preferably as follows:
d₁₀: ≤ 20 µm, preferably 2 to 10 µm
d₅₀: 5 to 100 µm, preferably 10 to 50 µm
d₉₀: 20 to 150 µm, preferably 40 to 100 µm

The tablet generally contains 0.1 to 20 mg, preferably 0.5 to 10.0 mg, of repaglinide and 100 to 3000 mg, preferably 200 to 1000 mg, of metformin hydrochloride.

In an even preferred embodiment, the disintegrating tablet matrix comprises a binder, a filler, a disintegrant and, optionally, other excipients and/or adjuvants.

The tablet composition according to the present invention generally comprises 5 to 95 wt.%, preferably 10 to 80 wt.%, of active ingredients; 0 to 20 wt.%, preferably 3 to 10 wt.%, of dry binder; 0 to 10 wt.%, preferably 1 to 5 wt.%, of wet granulation binder; 0 to 95 wt.%, preferably 20 to 90 wt.%, of filler and 0 to 50 wt.%, preferably 1 to 10 wt.%, of disintegrant.

The binder is selected from the group consisting of dry binders and/or wet granulation binders. Suitable dry binders are, e.g., cellulose powder and microcrystalline cellulose. Specific examples of wet granulation binders are corn starch, polyvinyl pyrrolidone (Povidon), vinylpyrrolidone-vinylacetate copolymer (Copovidone) and cellulose derivatives like hydroxymethylcellulose, hydroxyethylcellulose, hydroxypropylcellulose and hydroxyl-propylmethylcellulose.

The filler is preferably selected from anhydrous lactose, spray-dried lactose, mannitol, erythritol, sucrose, sorbitol, calcium phosphate, microcrystalline cellulose and lactose monohydrate.

Suitable disintegrants are, e.g., sodium starch glycolate, polacrilin potassium, Crospovidon, Croscarmellose, sodium carboxymethylcellulose and dried corn starch; sodium starch glycolate and polacrilin potassium being preferred.

The other excipients and/or adjuvants are, for instance, selected from carriers, lubricants, flow control agents, crystallization retarders, solubilizers, colouring agents, pH control agents, surfactants and emulsifiers, specific examples of which are given below. The excipients and/or adjuvants are preferably chosen such that a non-acidic, fast dissolving tablet matrix is obtained.

Other (optional) constituents may, for instance, be chosen from one or more of the following excipients and/or adjuvants in the amounts indicated:
0 to 10 wt.%, preferably 0.1 to 5 wt.%, of lubricants;
0 to 10 wt.%, preferably 1 to 5 wt.%, of flow control agents;
0 to 10 wt.%, preferably 0 to 0.5 wt.%, of colouring agents;

The other excipients and adjuvants, if used, are preferably selected from diluents and carriers such as cellulose powder, microcrystalline cellulose, cellulose derivatives like hydroxymethylcellulose, hydroxyethylcellulose, hydroxypropylcellulose and hydroxypropylmethylcellulose, dibasic calcium phosphate, corn starch, pregelatinized starch, polyvinyl pyrrolidone (Povidone) etc.; lubricants such as stearic acid, magnesium stearate, sodium stearylfumarate, glycerol tribehenate, etc.; flow control agents such as colloidal silica, talc, etc.; crystallization retarders such as Povidone, etc.; solubilizers such as Pluronic, Povidone, etc.; colouring agents, including dyes and pigments such as Iron Oxide Red or Yellow, titanium dioxide, talc, etc.; pH control agents such as citric acid, tartaric acid, fumaric acid, sodium citrate, dibasic calcium phosphate, dibasic sodium phosphate, etc.; surfactants and emulsifiers such as Pluronic, polyethylene glycols, sodium carboxymethyl cellulose, polyethoxylated and hydrogenated castor oil, etc.; and mixtures of two or more of these excipients and/or adjuvants.

The tablets obtained release the active ingredients rapidly and in a largely pH-independent fashion, with complete release occurring within less than 15 minutes and release of the major fraction occurring within less than 5 minutes.

In accordance with the present invention, a substantially increased dissolution rate of the active ingredients is achieved. Normally, at least 70% and typically at least 90% of the drug load are dissolved after 30 minutes.

The tablets of the present invention tend to be slightly hygroscopic and therefore are preferably packaged using a moisture-proof packaging material such as aluminium foil blister packs, or polypropylene tubes and HDPE bottles which preferably contain a desiccant.

In a second aspect, the present invention relates to a method of producing the pharmaceutical tablet according to the present invention comprising the steps of:
(a) preparing a granulate by granulating and drying a mixture of repaglinide and metformin with a binder solution, using the fluidized bed granulation process,
(b) mixing the granulate obtained in step (b) with a filler and a disintegrant,
(c) blending the mixture obtained in step (c) with other excipients and/or adjuvants and
(d) compression of the product obtained in step (d) into pharmaceutical tablets.

Repaglinide is preferably used in the form of a spray dried granulate or as an active triturate as mentioned hereinbefore; metformin is preferably used in the form of its hydrochloride salt with the specific size distribution as mentioned hereinbefore.

According to a further embodiment of the invention, the binder in step (a) is selected from the group consisting of dry binders and/or the group of wet granulation binders and is solved in purified water or a polar organic solvent, preferably ethanol or isopropanol. The solution thus obtained has a concentration of 0.1 to 30% by weight, preferably of 1 to 10% by weight.

Suitable dry binders are, e.g., cellulose powder and microcrystalline cellulose. Specific examples of wet granulation binders are corn starch, polyvinyl pyrrolidone (Povidon), vinylpyrrolidone-vinylacetate copolymer (Copovidone) and cellulose derivatives like hydroxymethylcellulose, hydroxyethylcellulose, hydroxypropylcellulose and hydroxyl-propylmethylcellulose.

The total amount of dry binder is so chosen as to be 0 to 20 wt.%, preferably 3 to 10 wt.%, related to the final tablet formulation.

The total amount of wet granulation binder is so chosen as to be 0 to 10 wt.%, preferably 1 to 5 wt.%, related to the final tablet formulation.

According to an even further embodiment the moisture content of the granulate obtained in step (a) is controlled to be between 0.1 to 1.5% after drying.

According to an even further embodiment the filler in step (b) is selected from the group consisting of anhydrous lactose, spray-dried lactose, mannitol, erythritol, sucrose, sorbitol, calcium phosphate, microcrystalline cellulose and lactose monohydrate.

The total amount of filler is so chosen as to be 0 to 95 wt.%, preferably 20 to 90 wt.%, related to the final tablet formulation.

According to an even further embodiment the disintegrant in step (b) is selected from the group consisting of sodium starch glycolate, polacrilin potassium, Crospovidon, Croscarmellose, sodium carboxymethylcellulose and dried corn starch; sodium starch glycolate and polacrilin potassium being preferred.

The total amount of disintegrant is so chosen as to be 0 to 50 wt.%, preferably 1 to 10 wt.%, related to the final tablet formulation.

According to an even further embodiment the amount of disintegrant in step (b) is from 1 to 500 mg, preferably from 10 to 100 mg, per tablet.

According to an even further embodiment the other excipients and/or adjuvants in step (c) are selected from the group consisting of carriers, lubricants, flow control agents, crystallization retarders, solubilizers, colouring agents, pH control agents, surfactants and emulsifiers, specific examples of which are given below. The excipients and/or adjuvants are preferably chosen such that a non-acidic, fast dissolving tablet matrix is obtained.

The other excipients and adjuvants, if used, are preferably selected from diluents and carriers such as cellulose powder, microcrystalline cellulose, cellulose derivatives like hydroxymethylcellulose, hydroxyethylcellulose, hydroxypropylcellulose and hydroxypropylmethylcellulose, dibasic calcium phosphate, corn starch, pregelatinized starch, polyvinyl pyrrolidone (Povidone) etc.; lubricants such as stearic acid, magnesium stearate, sodium stearylfumarate, glycerol tribehenate, etc.; flow control agents such as colloidal silica, talc, etc.; crystallization retarders such as Povidone, etc.; solubilizers such as Pluronic, Povidone, etc.; colouring agents, including dyes and pigments such as Iron Oxide Red or Yellow, titanium dioxide, talc, etc.; pH control agents such as citric acid, tartaric acid, fumaric acid, sodium citrate, dibasic calcium phosphate, dibasic sodium phosphate, etc.; surfactants and emulsifiers such as Pluronic, polyethylene glycols, sodium carboxymethyl cellulose, polyethoxylated and hydrogenated castor oil, etc.; and mixtures of two or more of these excipients and/or adjuvants.

The total amount of lubricant is so chosen as to be 0 to 10 wt.%, preferably 0.1 to 5 wt.%, related to the final tablet formulation.

The total amount of flow control agent is so chosen as to be 0 to 10 wt.%, preferably 1 to 5 wt.%, related to the final tablet formulation.

The total amount of colouring agent is so chosen as to be 0 to 10 wt.%, preferably 0 to 0.5 wt.%, related to the final tablet formulation.

According to an even further embodiment the hardness of the tablet obtained in step (d) is controlled to be between 20 and 300 N, preferably between 50 to 200 N.

For preparing the tablet according to the present invention, the tablet layer composition may be compressed in the usual manner in a monolayer tablet press, e.g. a high-speed rotary press in a bilayer or multilayer tabletting mode.

Although the monolayer tablet is the preferred form according to the present invention, it is also possible to prepare a bilayer or even multilayer, wherein the tablet layer composition may be compressed in the usual manner as mentioned above in a bilayer or multilayer tablet press.

For instance, the first tablet layer may be compressed at moderate force of 4 to 8 kN, whereas the main compression of first plus second layer is performed at a force of 10 to 300 kN, preferably 15 to 50 kN.

It was impossible to make tablets by the direct compression method due to the poor compressibility, even for a formulation which include 100 mg/tablet Povidone K25.

In the formulations according to the present invention, the moisture content of the granulate for tabletting should be between 1.5% and 3.0%. If it is lower than 1.5%, it is very difficult to make tablets due to poor compressibility. If it is higher than 3.0%, it is also very difficult due to poor flowability. Preferably, the moisture content should be between 1.8% and 2.5%.

In order to further illustrate the present invention, the following non-limiting examples are given.

### EXPERIMENTAL PART

### Example 1

Tablet containing 1.0 mg repaglinide and 650 mg metformin:

| | |
|---|---|
| Povidone K25 | 25.0 mg |
| Metformin HCl | 650.0 mg |
| Repaglinide triturate | 14.072 mg |
| Polacrilin potassium | 30.0 mg |
| Microcrystalline cellulose | 90.0 mg |
| Magnesium stearate | 5.0 mg |
| Total | 814.072 mg |

### Preparing procedure:

Povidone K25 was dissolved in purified water (granulation liquid). Metformin HCl and repaglinide triturate were charged into a suitable fluid bed granulator (e.g. WSG-5: Powrex Co., Ltd.), briefly pre-mixed and granulated by spraying granulation liquid. Thereafter, the granulate was screened using a suitable screening machine with mesh size of ca 0.5 mm. Screened granulate, microcrystalline cellulose and polacrilin potassium were mixed together using a suitable mixer. Then, magnesium stearate was added to the mixture and mixed using a suitable mixer (final mixture). The final mixture was compressed by a suitable tabletting machine.

### Example 2

Tablet containing 1.0 mg repaglinide and 650 mg metformin:

| | |
|---|---|
| Povidone K25 | 37.5 mg |
| Metformin HCl | 650.0 mg |
| Repaglinide triturate | 14.072 mg |
| Polacrilin potassium | 30.0 mg |
| Microcrystalline cellulose | 60.0 mg |
| Magnesium stearate | 5.0 mg |
| Total | 796.572 mg |

preparing procedure according to Example 1

### Example 3

Tablet containing 2.0 mg repaglinide and 650 mg metformin:

| | |
|---|---|
| Povidone K25 | 25.0 mg |
| Metformin HCl | 650.0 mg |
| Repaglinide triturate | 28.144 mg |
| Polacrilin potassium | 30.0 mg |
| Microcrystalline cellulose | 78.0 mg |
| Magnesium stearate | 5.0 mg |
| Total | 816.144 mg |

preparing procedure according to Example 1

### Example 4

Tablet containing 2.0 mg repaglinide and 650 mg metformin:

| | |
|---|---|
| Povidone K25 | 37.5 mg |
| Metformin HCl | 650.0 mg |
| Repaglinide triturate | 28.144 mg |
| Polacrilin potassium | 30.0 mg |
| Microcrystalline cellulose | 48.0 mg |
| Magnesium stearate | 5.0 mg |
| Total | 798.644 mg |

preparing procedure according to Example 1

### Example 5

Tablet containing 4.0 mg repaglinide and 650 mg metformin:

| | |
|---|---|
| Povidone K25 | 25.0 mg |
| Metformin HCl | 650.0 mg |
| Repaglinide triturate | 56.288 mg |
| Polacrilin potassium | 30.0 mg |
| Microcrystalline cellulose | 54.0 mg |
| Magnesium stearate | 5.0 mg |
| Total | 820.288 mg |

preparing procedure according to Example 1

### Example 6

Tablet containing 4.0 mg repaglinide and 650 mg metformin:

| | |
|---|---|
| Povidone K25 | 25.0 mg |
| Metformin HCl | 650.0 mg |
| Repaglinide triturate | 56.288 mg |
| Polacrilin potassium | 30.0 mg |
| Microcrystalline cellulose | 90.0 mg |
| Magnesium stearate | 5.0 mg |
| Total | 856.288 mg |

preparing procedure according to Example 1

### Example 7

Tablet containing 1.0 mg repaglinide and 500 mg metformin:

| | |
|---|---|
| Povidone K25 | 20.0 mg |
| Metformin HCl | 500.0 mg |
| Repaglinide triturate | 14.072 mg |
| Na-carboxymethylcellulose | 25.0 mg |
| Microcrystalline cellulose | 75.0 mg |
| Magnesium stearate | 2.0 mg |
| Total | 636.072 mg |

### Preparing procedure:

Povidone K25 was dissolved in purified water (granulation liquid). Metformin HCl and repaglinide triturate were charged into a suitable fluid bed granulator (e.g. WSG-5: Powrex Co., Ltd.), briefly pre-mixed and granulated by spraying granulation liquid. Thereafter, the granulate was screened using a suitable screen with mesh size of ca 0.5 mm. Screened granulate, microcrystalline cellulose and Na-carboxymethylcellulose were mixed together using a suitable mixer. Then, magnesium stearate was added to the mixture and mixed using a suitable mixer (final mixture). The final mixture was compressed by a suitable tabletting machine.

### Example 8

Tablet containing 2.0 mg repaglinide and 500 mg metformin:

| | |
|---|---|
| Povidone K25 | 20.0 mg |
| Metformin HCl | 500.0 mg |
| Repaglinide triturate | 28.144 mg |
| Na-carboxymethylcellulose | 25.0 mg |
| Microcrystalline cellulose | 75.0 mg |
| Magnesium stearate | 2.0 mg |
| Total | 650.144 mg |

preparing procedure according to Example 7

### Example 9

Tablet containing 3.0 mg repaglinide and 500 mg metformin:

| | |
|---|---|
| Povidone K25 | 20.0 mg |
| Metformin HCl | 500.0 mg |
| Repaglinide triturate | 42.216 mg |
| Na-carboxymethylcellulose | 25.0 mg |
| Microcrystalline cellulose | 75.0 mg |
| Magnesium stearate | 2.0 mg |
| Total | 664.216 mg |

preparing procedure according to Example 7

### Example 10

Tablet containing 1.0 mg repaglinide and 650 mg metformin:

| | |
|---|---|
| Povidone K25 | 20.0 mg |
| Metformin HCl | 650.0 mg |
| Repaglinide triturate | 14.072 mg |
| Na-carboxymethylcellulose | 25.0 mg |
| Microcrystalline cellulose | 75.0 mg |
| Magnesium stearate | 2.0 mg |
| Total | 786.072 mg |

preparing procedure according to Example 7

### Example 11

Tablet containing 1.0 mg repaglinide and 650 mg metformin:

| | |
|---|---|
| Povidone K25 | 50.0 mg |
| Metformin HCl | 650.0 mg |
| Repaglinide triturate | 14.072 mg |
| Na-carboxymethylcellulose | 25.0 mg |
| Microcrystalline cellulose | 75.0 mg |
| Magnesium stearate | 2.0 mg |
| Total | 816.072 mg |

preparing procedure according to Example 7

### Example 12

Tablet containing 1.0 mg repaglinide and 800 mg metformin:

| | |
|---|---|
| Povidone K25 | 50.0 mg |
| Metformin HCl | 800.0 mg |
| Repaglinide triturate | 14.072 mg |
| Na-carboxymethylcellulose | 50.0 mg |
| Microcrystalline cellulose | 100.0 mg |
| Magnesium stearate | 2.0 mg |
| Total | 1019.072 mg |

preparing procedure according to Example 7

### Example 13

Tablet containing 1.0 mg repaglinide and 800 mg metformin:

| | |
|---|---|
| Povidone K25 | 50.0 mg |
| Metformin HCl | 800.0 mg |
| Repaglinide triturate | 28.144 mg |
| Na-carboxymethylcellulose | 50.0 mg |
| Microcrystalline cellulose | 100.0 mg |
| Magnesium stearate | 2.0 mg |
| Total | 1033.144 mg |

preparing procedure according to Example 7

### Example 14

Tablet containing 2.0 mg repaglinide and 500 mg metformin:

| | |
|---|---|
| Hydroxypropylcellulose | 20.0 mg |
| Metformin HCl | 500.0 mg |
| Repaglinide triturate | 28.144 mg |
| Na-carboxymethylcellulose | 25.0 mg |
| Microcrystalline cellulose | 75.0 mg |
| Magnesium stearate | 2.0 mg |
| Total | 650.144 mg |

### Preparing procedure:

Hydroxypropylcellulose was dissolved in purified water (granulation liquid). Metformin HCl and repaglinide triturate were charged into a suitable fluid bed granulator (e.g. WSG-5: Powrex Co., Ltd.), briefly pre-mixed and granulated by spraying granulation liquid. Thereafter, the granulate was screened using a suitable screen with mesh size of ca 0.5 mm. Screened granulate, microcrystalline cellulose and Na-carboxymethylcellulose were mixed together using a suitable mixer. Then, magnesium stearate was added to the mixture and mixed using a suitable mixer (final mixture). The final mixture was compressed by a suitable tabletting machine.

### Example 15

Tablet containing 2.0 mg repaglinide and 500 mg metformin:

| | |
|---|---|
| Hydroxypropylcellulose | 20.0 mg |
| Metformin HCl | 500.0 mg |
| Repaglinide triturate | 28.144 mg |
| Crospovidon | 25.0 mg |
| Microcrystalline cellulose | 50.0 mg |
| Lactose | 100.0 mg |
| Magnesium stearate | 2.0 mg |
| Total | 650.144 mg |

### Preparing procedure:

Hydroxypropylcellulose was dissolved in purified water (granulation liquid). Metformin HCl, repaglinide triturate and lactose were charged into a suitable fluid bed granulator (e.g. WSG-5: Powrex Co., Ltd.), briefly pre-mixed and granulated by spraying granulation liquid. Thereafter, the granulate was screened using a suitable screen with mesh size of ca 0.5 mm. Screened granulate, microcrystalline cellulose and Crospovidone were mixed together using a suitable mixer. Then, magnesium stearate was added to the mixture and mixed using a suitable mixer (final mixture). The final mixture was compressed by a suitable tabletting machine.

### Example 16

Tablet containing 2.0 mg repaglinide and 500 mg metformin:

| | |
|---|---|
| Hydroxypropylcellulose | 20.0 mg |
| Metformin HCl | 500.0 mg |
| Repaglinide triturate | 28.144 mg |
| Croscarmellose | 25.0 mg |
| Microcrystalline cellulose | 50.0 mg |
| Lactose | 100.0 mg |
| Magnesium stearate | 2.0 mg |
| Total | 650.144 mg |

### Preparing procedure:

Hydroxypropylcellulose was dissolved in purified water (granulation liquid). Metformin HCl, repaglinide triturate and lactose were charged into a suitable fluid bed granulator (e.g. WSG-5: Powrex Co., Ltd.), briefly pre-mixed and granulated by spraying granulation liquid. Thereafter, the granulate was screened using a suitable screen with mesh size of ca 0.5 mm. Screened granulate, microcrystalline cellulose and croscarmellose were mixed together using a suitable mixer. Then, magnesium stearate was added to the mixture and mixed using a suitable mixer (final mixture). The final mixture was compressed by a suitable tabletting machine.

### Example 17

Tablet containing 2.0 mg repaglinide and 500 mg metformin:

| | |
|---|---|
| Hydroxypropylcellulose | 20.0 mg |
| Metformin HCl | 500.0 mg |
| Repaglinide triturate | 28.144 mg |
| Croscarmellose | 25.0 mg |
| Microcrystalline cellulose | 50.0 mg |
| Mannitol | 100.0 mg |
| Magnesium stearate | 2.0 mg |
| Total | 650.144 mg |

### Preparing procedure:

Hydroxypropylcellulose was dissolved in purified water (granulation liquid). Metformin HCl, repaglinide triturate and mannitol were charged into a suitable fluid bed granulator (e.g. WSG-5: Powrex Co., Ltd.), briefly pre-mixed and granulated by spraying granulation liquid. Thereafter, the granulate was screened using a suitable screen with mesh size of ca 0.5 mm. Screened granulate, microcrystalline cellulose and Croscarmellose were mixed together using a suitable mixer. Then, magnesium stearate was added to the mixture and mixed using a suitable mixer (final mixture). The final mixture was compressed by a suitable tabletting machine.

### Example 18

Tablet containing 2.0 mg repaglinide and 500 mg metformin:

| | |
|---|---|
| Hydroxypropylcellulose | 20.0 mg |
| Metformin HCl | 500.0 mg |
| Repaglinide triturate | 28.144 mg |
| Croscarmellose | 25.0 mg |
| Microcrystalline cellulose | 50.0 mg |
| Calcium phosphate | 100.0 mg |
| Magnesium stearate | 2.0 mg |
| Total | 650.144 mg |

### Preparing procedure:

Hydroxypropylcellulose was dissolved in purified water (granulation liquid). Metformin HCl, repaglinide triturate and calcium phosphate were charged into a suitable fluid bed granulator (WSG-5: Powrex Co., Ltd.), briefly pre-mixed and granulated by spraying granulation liquid. Thereafter, the granulate was screened using a suitable screen with mesh size of ca 0.5 mm. Screened granulate, microcrystalline cellulose and Croscarmellose were mixed together using a suitable mixer. Then, magnesium stearate was added to the mixture and mixed using a suitable mixer (final mixture). The final mixture was compressed by a suitable tabletting machine.

### Example 19

Tablet containing 2.0 mg repaglinide and 500 mg metformin:

| | |
|---|---|
| Hydroxypropylcellulose | 20.0 mg |
| Metformin HCl | 500.0 mg |
| Repaglinide triturate | 28.144 mg |
| Croscarmellose | 25.0 mg |
| Microcrystalline cellulose | 50.0 mg |
| Calcium phosphate | 100.0 mg |
| Magnesium stearate | 5.0 mg |
| Total | 653.144 mg |

preparing procedure according to Example 18

### Example 20

Tablet containing 3.0 mg repaglinide and 650 mg metformin:

| | |
|---|---|
| Povidone K25 | 25.0 mg |
| Metformin HCl | 650.0 mg |
| Repaglinide triturate | 42.216 mg |
| Polacrilin potassium | 30.0 mg |
| Microcrystalline cellulose | 70.0 mg |
| Magnesium stearate | 5.0 mg |
| Total | 822.216 mg |

Preparing procedure according to Example 1

## Claims

1. Process for preparing a pharmaceutical tablet comprising repaglinide and metformin in a disintegrating tablet matrix said method comprising the steps of
(a) preparing a granulate by granulating and drying a mixture of repaglinide and metformin with a binder solution, using the fluidized bed granulation process, wherein repaglinide is used in the form of a spray dried granulate or an active triturate,
(b) mixing the granulate obtained in step (b) with a filler and a disintegrant,
(c) blending the mixture obtained in step (c) with other excipients and/or adjuvants and
(d) compression of the product obtained in step (d) into pharmaceutical tablets.

2. The process according to claim 1, wherein in step (a) metformin is used in the form of its hydrochloride salt with the following particle size distribution:
d₁₀: ≤ 20 µm, preferably 2 to 10 µm
d₅₀ : 5 to 100 µm, preferably 10 to 50 µm
d₉₀ : 20 to 150 µm, preferably 40 to 100 µm.

3. The process according to claim 1, wherein the binder is selected from the group consisting of dry binders and/or wet granulation binders.

4. The process according to claim 3, wherein the dry binder is selected from the group consisting of cellulose powder and microcrystalline cellulose.

5. The process according to claim 3, wherein the total amount of dry binder is so chosen as to be 0 to 20 wt.%, preferably 3 to 10 wt.%, related to the final tablet formulation.

6. The process according to claim 3, wherein the wet granulation binder is selected from the group consisting of corn starch, polyvinyl pyrrolidone (Povidon), vinylpyrrolidone-vinylacetate copolymer (Copovidone) and cellulose derivatives like hydroxymethylcellulose, hydroxyethylcellulose, hydroxypropylcellulose and hydroxylpropylmethyl-cellulose.

7. The process according to claim 3, wherein the total amount of wet granulation binder is so chosen as to be 0 to 10 wt.%, preferably 1 to 5 wt.%, related to the final tablet formulation.

8. The process according to claim 1, wherein the binder is solved in purified water or a polar organic solvent.

9. The process according to claim 1, wherein the polar organic solvent is ethanol or isopropanol.

10. The process according to claim 1, wherein the binder solution has a concentration of 0.1 to 30% by weight.

11. The process according to claim 1, wherein in step (a) the moisture content of the granulate is controlled to be between 0.1 to 1.5 % after drying.

12. The process according to claim 1, wherein the filler in step (b) is selected from the group consisting of anhydrous lactose, spray-dried lactose, mannitol, erythritol, sucrose, sorbitol, calcium phosphate, microcrystalline cellulose and lactose monohydrate.

13. The process according to claim 12, wherein the total amount of filler is so chosen as to be 0 to 95 wt.%, preferably 20 to 90 wt.%, related to the final tablet formulation.

14. The process according to claim 1, wherein the disintegrant in step (b) is selected from the group consisting of sodium starch glycolate, polacrilin potassium, Crospovidon, Croscarmellose, sodium carboxymethylcellulose and dried corn starch.

15. The process according to claim 14, wherein the total amount of disintegrant is so chosen as to be 0 to 50 wt.%, preferably 1 to 10 wt.%, related to the final tablet formulation.

16. The process according to claim 1, wherein the other excipients and/or adjuvants in step (c) are selected from the group consisting of carriers, lubricants, flow control agents, crystallization retarders, solubilizers, colouring agents, pH control agents, surfactants and emulsifiers.

17. The process according to claim 1, wherein in step (d) the hardness of the tablets is controlled to be between 20 N to 300 N.

## Patentansprüche

1. Verfahren zur Herstellung einer pharmazeutischen Tablette, umfassend Repaglinid und Metformin in einer sich zersetzenden Tablettenmatrix, wobei das Verfahren die folgenden Schritte umfasst:
a) Herstellen eines Granulats durch Granulieren und Trocknen eines Gemischs aus Repaglinid und Metformin mit einer Bindemittellösung unter Verwendung des Wirbelschichtgranulierungsverfahrens, wobei Repaglinid in der Form eines sprühgetrockneten Granulats oder einer aktiven Verreibung verwendet wird,
b) Mischen des in Schritt (a) erhaltenen Granulats mit einem Füllstoff und einem Sprengmittel,
c) Vermischen des in Schritt (b) erhaltenen Gemischs mit anderen Exzipienten und/oder Hilfsstoffen und
d) Pressung des in Schritt (c) erhaltenen Produkts zu pharmazeutischen Tabletten.

2. Verfahren nach Anspruch 1, wobei in Schritt (a) Metformin in der Form seines Hydrochloridsalzes mit der folgenden Teilchengrößenverteilung verwendet wird:
d₁₀: ≤ 20 µm, vorzugsweise 2 bis 10 µm
d₅₀: 5 bis 100 µm, vorzugsweise 10 bis 50 µm
d₉₀: 20 bis 150 µm, vorzugsweise 40 bis 100 µm

3. Verfahren nach Anspruch 1, wobei das Bindemittel ausgewählt ist aus der Gruppe, bestehend aus Trockenbindemitteln und/oder Nassgranulierungsbindemitteln.

4. Verfahren nach Anspruch 3, wobei das Trockenbindemittel ausgewählt ist aus der Gruppe, bestehend aus Cellulosepulver und mikrokristalliner Cellulose.

5. Verfahren nach Anspruch 3, wobei die Gesamtmenge an Trockenbindemittel derart ausgewählt ist, dass sie 0 bis 20 Gew.-%, vorzugsweise 3 bis 10 Gew.-% in Bezug auf die endgültige Tablettenformulierung beträgt.

6. Verfahren nach Anspruch 3, wobei das Nassgranulierungsbindemittel ausgewählt ist aus der Gruppe, bestehend aus Maisstärke, Polyvinylpyrrolidon (Povidon), Vinylpyrrolidon-Vinylacetat-Copolymer (Copovidon) und Cellulosederivaten wie Hydroxymethylcellulose, Hydroxyethylcellulose, Hydroxypropylcellulose und Hydroxypropylmethylcellulose.

7. Verfahren nach Anspruch 3, wobei die Gesamtmenge an Nassgranulierungsbindemittel derart ausgewählt ist, dass sie 0 bis 10 Gew.-%, vorzugsweise 1 bis 5 Gew.-% in Bezug auf die endgültige Tablettenformulierung beträgt.

8. Verfahren nach Anspruch 1, wobei das Bindemittel in gereinigtem Wasser oder in einem polaren organischen Lösungsmittel gelöst ist.

9. Verfahren nach Anspruch 1, wobei das polare organische Lösungsmittel Ethanol oder Isopropanol ist.

10. Verfahren nach Anspruch 1, wobei die Bindemittellösung eine Konzentration von 0,1 bis 30 Gew.-% aufweist.

11. Verfahren nach Anspruch 1, wobei in Schritt (a) der Feuchtigkeitsgehalt des Granulats derart gesteuert wird, dass er nach dem Trocknen zwischen 0,1 bis 1,5% beträgt.

12. Verfahren nach Anspruch 1, wobei der Füllstoff in Schritt (b) ausgewählt ist aus der Gruppe, bestehend aus wasserfreier Lactose, sprühgetrockneter Lactose, Mannit, Erythritol, Saccharose, Sorbit, Calciumphosphat, mikrokristalliner Cellulose und Lactosemonohydrat.

13. Verfahren nach Anspruch 12, wobei die Gesamtmenge an Füllstoff derart ausgewählt ist, dass sie 0 bis 95 Gew.-%, vorzugsweise 20 bis 90 Gew.-% in Bezug auf die endgültige Tablettenformulierung beträgt.

14. Verfahren nach Anspruch 1, wobei das Sprengmittel in Schritt (b) ausgewählt ist aus der Gruppe, bestehend aus Natriumstärkeglycolat, Polacrilinkalium, Crospovidon, Croscarmellose, Natriumcarboxymethylcellulose und getrockneter Maisstärke.

15. Verfahren nach Anspruch 14, wobei die Gesamtmenge an Sprengmittel derart ausgewählt ist, dass sie 0 bis 50 Gew.-%, vorzugsweise 1 bis 10 Gew.-% in Bezug auf die endgültige Tablettenformulierung beträgt.

16. Verfahren nach Anspruch 1, wobei die anderen Exzipienten und/oder Hilfsstoffe in Schritt (c) ausgewählt sind aus der Gruppe, bestehend aus Trägern, Schmiermitteln, Flusssteuerungsmitteln, Kristallisationsverzögerern, Löslichmachern, Farbmitteln, pH-Steuerungsmitteln, oberflächenaktiven Mitteln und Emulgatoren.

17. Verfahren nach Anspruch 1, wobei in Schritt (d) die Härte der Tabletten derart gesteuert wird, dass sie zwischen 20 N bis 300 N beträgt.

## Revendications

1. Procédé de préparation d'un comprimé pharmaceutique comprenant du répaglinide et de la metformine dans une matrice pour comprimé délitable, ledit procédé comprenant les étapes de
(a) préparation d'un granulé par granulation et séchage d'un mélange de répaglinide et de metformine avec une solution liante, par utilisation du procédé de granulation en lit fluidisé, le répaglinide étant utilisé sous forme d'un granulé séché par atomisation ou d'un triturat actif,
(b) mélange du granulé obtenu dans l'étape (b) avec une charge et un désintégrant,
(c) mélange du mélange obtenu dans l'étape (c) avec d'autres excipients et/ou adjuvants, et
(d) compression du produit obtenu dans l'étape (d), pour donner des comprimés pharmaceutiques.

2. Procédé selon la revendication 1, dans lequel, dans l'étape (a), la metformine est utilisée sous forme de son sel chlorhydrate, avec la distribution granulométrique suivant :
d₁₀ : ≤ 20 µm, de préférence 2 à 10 µm
d₅₀ : 5 à 100 µm, de préférence 10 à 50 µm
d₉₀ : 20 à 150 µm, de préférence 40 à 100 µm.

3. Procédé selon la revendication 1, dans lequel le liant est choisi dans le groupe consistant en les liants secs et/ou les liants pour granulation par voie humide.

4. Procédé selon la revendication 3, dans lequel le liant sec est choisi dans le groupe consistant en la poudre de cellulose et la cellulose microcristalline.

5. Procédé selon la revendication 3, dans lequel la quantité totale du liant sec est choisie de façon à être de 0 à 20 % en poids, de préférence de 3 à 10 % en poids, par rapport à la formulation du comprimé final.

6. Procédé selon la revendication 3, dans lequel le liant pour granulation par voie humide est choisi dans le groupe consistant en l'amidon de maïs, la polyvinylpyrrolidone (Povidone), le copolymère vinylpyrrolidone-acétate de vinyle (Copovidone) et les dérivés de la cellulose tels que l'hydroxyméthylcellulose, l'hydroxyéthylcellulose, l'hydroxypropylcellulose et l'hydroxypropylméthyl-cellulose.

7. Procédé selon la revendication 3, dans lequel la quantité totale du liant pour granulation par voie humide est choisie de façon à être de 0 à 10 % en poids, de préférence de 1 à 5 % en poids, par rapport à la formulation du comprimé final.

8. Procédé selon la revendication 1, dans lequel le liant est dissous dans de l'eau purifiée ou dans un solvant organique polaire.

9. Procédé selon la revendication 1, dans lequel le solvant organique polaire est l'éthanol ou l'isopropanol.

10. Procédé selon la revendication 1, dans lequel la solution liante a une concentration de 0,1 à 30 % en poids.

11. Procédé selon la revendication 1, dans lequel, dans l'étape (a), la teneur en humidité des granulés est ajustée à une valeur comprise entre 0,1 et 1,5 % après séchage.

12. Procédé selon la revendication 1, dans lequel la charge de l'étape (b) est choisie dans le groupe consistant en le lactose anhydre, le lactose séché par atomisation, le mannitol, l'érythritol, le saccharose, le sorbitol, le phosphate de calcium, la cellulose monocristalline et le lactose monohydraté.

13. Procédé selon la revendication 12, dans lequel la quantité totale de la charge est choisie de façon à être de 0 à 95 % en poids, de préférence de 20 à 90 % en poids, par rapport à la formulation du comprimé final.

14. Procédé selon la revendication 1, dans lequel le désintégrant de l'étape (b) est choisi dans le groupe consistant en l'amidon-glycolate de sodium, la polacriline-potassium, la Crospovidone, la Croscarmellose, la carboxyméthylcellulose sodique et l'amidon de maïs séché.

15. Procédé selon la revendication 14, dans lequel la quantité totale du désintégrant est choisie de façon à être de 0 à 50 % en poids, de préférence de 1 à 10 % en poids, par rapport à la formulation du comprimé final.

16. Procédé selon la revendication 1, dans lequel les autres excipients et/ou adjuvants de l'étape (c) sont choisis dans le groupe consistant en les supports, les lubrifiants, les agents régulateurs d'écoulement, les retardateurs de cristallisation, les solubilisateurs, les agents colorants, les agents régulateurs de pH, les tensioactifs et les émulsifiants.

17. Procédé selon la revendication 1, dans lequel, dans l'étape (d), la dureté des comprimés est régulée à une valeur comprise entre 20 et 300 N.
